# EUROPEAN PATENT SPECIFICATION

(11) **EP 0 531 475 B1**
(45) Date of publication and mention of the grant of the patent: **28.08.2002**
(21) Application number: 92906378.2
(22) Date of filing: 09.03.1992
(51) Int. Cl.: C07C 255/54, C09K 19/32, C09K 19/34, C07C 321/24, C07C 331/28, C07D 239/26, C07D 333/24, C07D 333/36, G02F 1/13

(54) **NAPHTHYL ORGANIC COMPOUNDS**
ORGANISCHE NAPHTYL VERBINDUNGEN
COMPOSES ORGANIQUES NAPHTYLIQUES

(30) Priority: 13.03.1991 GB 9105359
(43) Date of publication of application: 17.03.1993
(73) Proprietor: QinetiQ Limited, London, SW1 6TD (GB)
(72) Inventor: TOYNE, Kenneth Johnson, Humberside HU6 7RX (GB); GOODBY, John William, Humberside HU6 7RX (GB); SEED, Alexander, Humberside HU6 7RX (GB); GRAY, George William, Wimborne, Dorset BH21 4HD (GB); McDONNELL, Damien Gerard, Malvern, Worcestershire WR14 3PS (GB); RAYNES, Edward Peter, Malvern, Worcestershire WR14 3PS (GB); DAY, Sally Elizabeth, Malvern, Worcestershire WR14 3PS (GB); HARRISON, Kenneth John, Malvern, Worcestershire WR14 3PS (GB); HIRD, Michael, Humberside HU6 7RX (GB)
(86) International application number: GB9200411
(87) International publication number: WO92016500

(56) References cited:
- EP-A- 0 341 922
- WO-A-87/06577
- GB-A- 1 603 075
- GB-A- 2 070 593
- Patent Abstracts of Japan, vol. 10, no. 328, (C-383)[2384], 7th November 1986; & JP, A, 61134364 (CHISSO CORP.) 21 June 1986, see abstract

## Description

This invention relates to compounds containing a naphthyl group and which have liquid crystalline properties and/or which are suitable for use as constituents of liquid crystal materials. The invention also relates to use of such compounds in liquid crystal materials.

Liquid crystal materials and devices exploit the electro-optical propertiesof nematic and cholesteric (N or N°), or smectic (S) with particularly useful smectic phases being chiral smectic C (S_{C}*) or smectic A.

Liquid crystal materials which show ferroelectric S_{C}* phase are useful in fast switching displays such as television or VDU screens as the S_{C}* phase can be swithched in a few milliseconds or even microseconds. The principle of S_{C}* switching is described *inter alia* by N A Clark and S T Lagerwall in App Phys Lett 36 (1980) p899.

Materials which show an S_{A} liquid crystal phase may be used in display devices which exploit the electroclinic effect.

The use of liquid crystal materials to exhibit electro-optical effects in displays and other optical devices such as digital calculators, watches, meters and alphanumeric laptop computers is now well known. However, known liquid crystal materials are not ideal in all respects and a considerable amount of work is currently being carried out in the art to improve their properties.

Liquid crystal materials normally consist of specially selected mixture compositions and improved materials are often obtained by forming new mixtures having an improved combination of properties.

The composition of a liquid crystal mixture is generally selected so that the mixture shows desirable properties. In particular such properties include:
(1) a liquid crystalline temperature range - including room temperature (20°C) - which is as wide as possible;
(2) a melting point (solid-to-liquid crystal transition temperature) which is as low as possible;
(3) a clearing point (liquid crystalline to isotropic liquid transition temperature) which is as high as possible;
(4) a positive or negative (as appropriate) dielectric anisotropy (permittivity measured parallel to the molecular axis less that measured perpendicular to the molecular axis) which is as great as possible in order to minimise the display voltage;
(5) a viscosity which is as low as possible in order to minimise the display switching speeds;
(6) an electro-optical response which varies as little as possible with temperatures;
(7) a good chemical and photochemical stability;
   Examples of further particular properties useful in specific applications include:
(8) a good multiplexability;
(9) an ability to switch dielectric anisotropy with frequency: (10) a birefringence of selected magnitude;
(11) specific elastic constants which can be tailored to meet specific device requirements; and
(12) high electrical resistivity for certain applications.
Liquid crystal materials and devices exploit the electro-optical properties of nematic or cholesteric (N and N^{*} respectively), or smectic (S) in particular chiral smectic C (S_{c}^{*}) or smectic A (Sₐ) phase. The most common type of liquid crystal materials in use are those which show a nematic phase and these are extensively used in, for example, watches, clocks, calculators, electronic displays etc.

For some applications of liquid crystal materials a high birefringence is sought, eg in so called "electrically controlled birefringence" (ECB) effect devices (see, for example. M F Schieckel and K Fahrensohn Applied Physics Letters 19 p2912 1971), in thin film transistors and supertwisted nematic devices. It is rare for the requisite desirable properties to be found in a single liquid crystalline compound and generally liquid crystalline materials consist of mixtures of component compounds. Very many liquid crystalline compounds suitable for such uses are known and will be apparent to those in the art.

Some liquid crystalline compounds based on the phenyl-naphthalene system are known e.g.

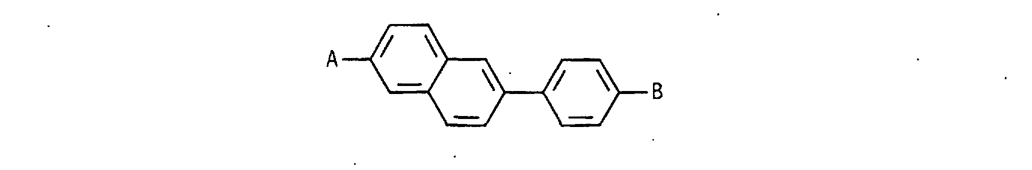

For example, Bull Soc Chim Fr 11-12(2) p2521-2426 (1975) describes compounds where A is alkoxy and B is alkyl or alkoxy, and Helv. Chim Acta 68(5) p1406-1426 describes those in which A is alkyl or alkoxy and B is cyano or trifluoromethyl.

It is the aim of this invention to provide novel compounds which provide improved or alternative liquid crystalline and/or monotropic characteristics.

WO - A-8706577 discloses alkoxy naphthyl containing compounds.

According to this invention, liquid crystal naphthyl compounds of Formula 1 are provided;

R₁―A―(X)ₘ―(B)ₙ―R₂ Formula I

where A is naphthyl, B is selected from phenyl, methylated phenyl, brominated phenyl, fluorinated phenyl, thiophene, pyrimidine and pyridine, R₁ is thioalkyl, R₂ is selected from CN, SCN, NCS, X is COO, m is 1, n is 1.

The preferred embodiments of the invention discussed below are *inter alia chosen* with respect to their liquid crystalline properties, particularly with respect to suitability for use in high birefringence nematic or ferroelectric S_{c}^{*} liquid crystal materials. Preferably for use in nematic materials thioalkyl substituent R₁ contains 1-15 carbon atoms, and more preferably contains 1-5 carbon atoms for use as nematic materials and 3-9 carbon atoms for use as smectic materials.

According to a further aspect of this invention compounds of formula I are suitable for inclusion in devices utilising pretransitional characteristics of liquid crystalline materials in the isotropic phase.

Typically such compounds can be utilised in devices such as Optical Kerr Effect devices. Such devices are often used as optical shutters or optical modulators, and rely on the fact that birefringence (Δn) of a medium being proportional to the square of an applied electric field. Such an effect is often termed the quadratic electro-optic effect and can be investigated using degenerate four wave mixing (P Madden et al IEEE J of Quantum Electronics QE22 No 8 Aug 1986 p1287).

Preferably R₁ is selected from C₁₋₁₅ thioalkyl more preferably C₁₋₉ and even more preferably C₃₋₉.

Naphthyl materials of formula I may be generally prepared by various routes which will be apparent to those in the art. Typical routes which can be used include reaction of an appropriate phenyl (or equivalent e.g. thiophene, pyrimidine etc) boronic acid with an appropriate naphthol triflate. Alternatively, an appropriate boronic acid can be reacted with such appropriate compounds as bromonitro - e.g. acid can be reacted with such appropriate compounds as bromonitro- e.g. appropriate benzene, thiophene residues etc, for CN and NCS terminal groups. Terminal groups of thioalkyls can be achieved by well known thioalkylation routes respectively.

The invention also provides a liquid crystal material being a mixture of at least two compounds, at least one of which is a naphthyl compound of formula I. This material may show a nematic or smectic (eg Sₐ, S_{c}* or S_{c}) charateristics and also sometimes isotropic properties.

In particular many naphthyl compounds of formula I have a high anisotropy in polarisability (Δa), making them suitable for applications in which this characteristic is required, as discussed above. Naphthyl compounds of formula I may be used as components of nematic liquid crystal materials together with other liquid crystalline compounds, for example compounds of general formula III: 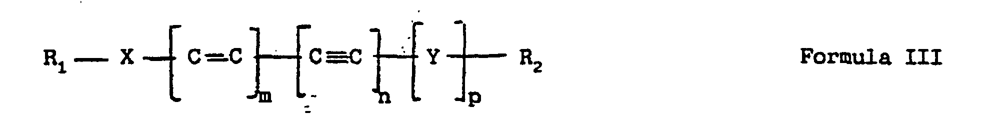 where R₁ is selected from a group comprising hydrogen, alkyl, alkoxy, alkynyl, thioalkyl, CN, and Br; R₂ is selected from a group comprising hydrogen, NCS, SCN, CN, alkyl, alkoxy, alkynyl, and thioalkyl; m and n are 1 or 0 such that m is 1 where n is 0 and m is 0 where n is 1 or 0; p is independently 1 or 0; X is selected from a group comprising of naphthyl, fluorinated naphthyl and brominated naphthyl; and Y is selected from a group comprising of phenyl, methylated phenyl, brominated phenyl, thiophene and pyrimidine and pyridine.

The mixture may also contain such materials as phenyl thiazolenes of typical structure such as that seen in Formula IV 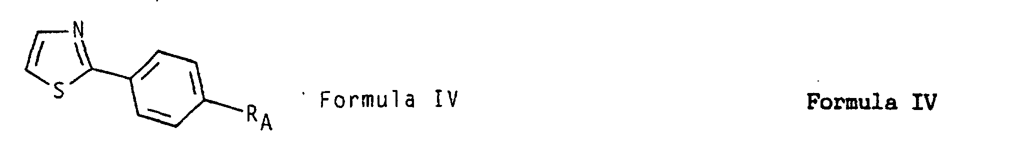 where typically R_{A} is CN, alkyl, alkoxy etc.

Other suitable mixture materials include for example those of materials described in Application Number 892828.6. where their use as polymer network materials suitable for eg dispersion within a matrix of transparent polymer is described.

The mixture may also contain one or more fluorinated cyanobiphenyls or terphenyls of formula V: 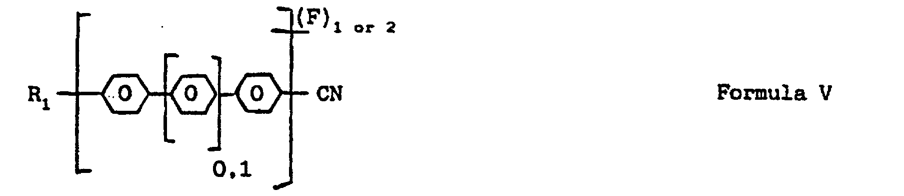 where R₁ is C₁₋₁₀ n-alkyl or n-alkoxyl and the fluorosubstituent(s) may be in any one or two of the available substitution positions. Compounds of the formula V are known (PCT/GB 89/00647) and with their inclusion in mixture, it is possible to further increase birefringence.

A nematic liquid crystal of this invention may also contain one or more optically active compounds to induce a cholestric phase and one or more pleochroic dyes.

The materials of this aspect of the invention may be used in any of the known forms of liquid crystal display devices, for example a twisted nematic device, Freedricks effect device, cholesteric memory mode device, cholesteric to nematic phase change effect device, dynamic scattering effect device, two frequency switching effect device, a supertwist effect device, or a thermometer using a thermochromic material. The method of construction and operation of such devices, and characteristics of a liquid crystal material suitable for use therein, are well known in the field. Typically an electro-optical display will consist of 2 substrates between which a layer of the liquid crystal material may be sandwiched. At least one of the substrates is optically transparent and both have addressable electrodes which are preferably made of a transparent material on their opposing faces. By applying an electric field across the layer of liquid crystal material via the electrodes an electro-optical effect is achieved which may be viewed directly or preferably through one or more polarising filters.

Another aspect of the invention is a material, being a mixture of compounds, characterised in that the mixture is suitable for inclusion in devices utilising pretransitional characterisitcs of liquid crystalline mixtures in the isotropic phase and that the mixture includes at least one compound of formula II. Such materials also contain compound(s) of formula III and/or compound(s) of formula IV and/or compound(s) of formula V.

Compounds of formula II and also materials including compound(s) of formula II may be used in devices that utilise the optical Kerr Effect. Typically optical Kerr effect devices comprise a glass cell containing two electrodes, where the glass cell is filled with a polar liquid. The device is frequently termed a Kerr cell. The Kerr cell can be positioned between two crossed polarisers having transmisssion axes at ±45° to an electric field applied across the Kerr cell. With zero voltage applied across the Kerr cell no light will be transmitted and the cell operates as a closed shutter. Application of a modulating voltage generates a field causing the Kerr cell to function as a variable- wave plate and thus operating the Kerr cell as a shutter capable of opening proportionately to the applied field.

Nematic materials of this invention may be particularly suitable for use in ECB effect devices, due to the high birefringence of the materials. They may also be particularly suitable for use in polymer dispersed liquid crystal (PDLC) materials in which small droplets of a liquid crystal material are dispersed within a matrix of a transparent polymer.

Non-limiting examples illustrating this invention will now be described with reference to the accompanying figures, of which figures 1 to 5 schematically show synthetic routes for exemplified compounds 1 to 5, and figures 6 and 7 are schematic representations of a liquid crystal device of the invention and a Kerr cell of the invention.

### Example 1. (Comparative) Preparation of:

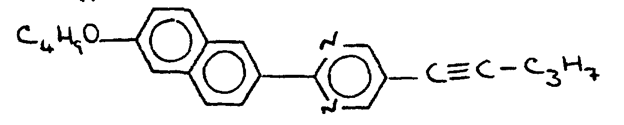

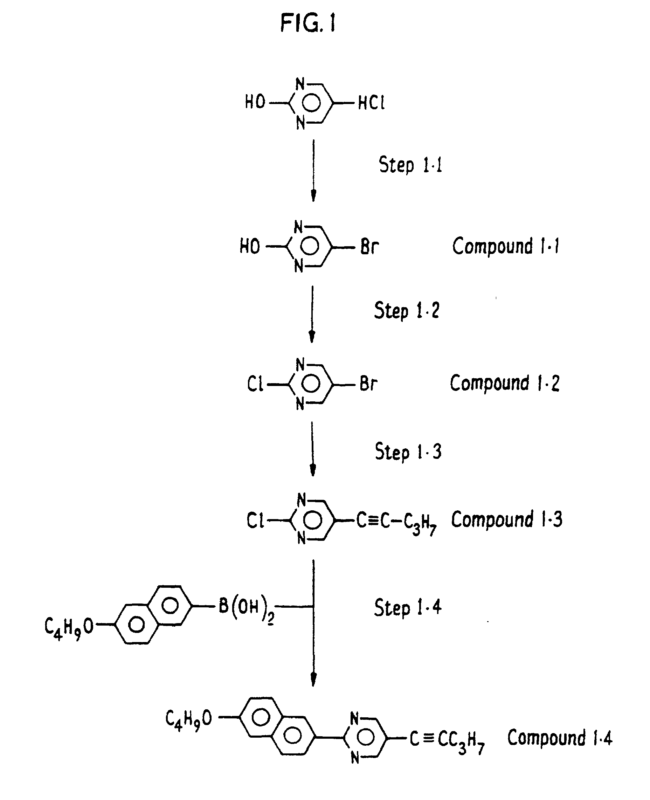

With reference to figure 1 it can be seen that
Step 1.1 5-Bromo-hydroxypyrimidine.
   Bromine (67.00g, 0.419mol) is added slowly dropwise to a stirred solution of 2-hydroxypyrimidine hydrochloride (50.00g, 0.377mol) in water (200ml) at room temperature (exothermic reaction but no cooling used). The solution is stirred for 1 hour (until cool) and then water and excess bromine is removed *in vacuo* to give a pale yellow solid which is dried *in vacuo* (0.1 mmHg) giving 5-Bromo-2-hydroxypyrimidine as a solid which is still "wet" as a yield of 100g.
Step 1.2 5-Bromo-2-chloropyrimidine.
   A solution of compound 1.1 (assume 100% yield from previous preparation; 65.98g, 0.377mol) in phosphorous oxychloride (500ml) and *N,N*-dimethylaniline (20ml) is heated under reflux for 4 hours, the cooled mixture is carefully poured onto ice and extracted into ether (x2). The combined ethereal extracts are washed with aqueous sodium hydrogen carbonate and dried (MgSO₄). The solvent is removed *in vacuo* to yield an off-white solid. Yield (based on 2-hydroxypyrimidine hydrochloride) is 23.55g, 32%.
Step 1.3 2-Chloro-5-pent-1-ynylpyrimidine.
   Quantities: pent-1-yne (3.00g, 0.044mol), n-butyllithium (4.40ml, 10.0M in hexane, 0.044mol), zinc chloride (6.00g, 0.044mol), compound 1.2 (8.00g, 0.041mol), tetrakis (triphenylphosphine)palladium(0) (1.5g, 1.30mol).
   This experimental procedure is a zinc coupling reaction. The n-butyllithium solution is added dropwise to a stirred, cooled (-5°C to 0°C) solution of the pent-1-yne in dry THF under dry nitrogen. This mixture is stirred fro 10 minutes and then a solution of the zinc chloride (dry) in dry THF is added dropwise at about -5°C to 0°C. The mixture is stirred at room temperature for 15 minutes and a solution of compound 1.2 in dry THF is added dropwise at -5°C to 0°C followed by addition of the tetrakis (triphenylphosphine)palladium(0). the mixture is heated under reflux for 22 hours (glc analysis revealing a complete reaction). The crude product is purified by column chromotography [silica gel/petroleum fraction (bp40-60°C)- dichloromethane, 1:20] to give a colourless solid which is crystalised from hexane to yield colourless crystals.Yield is 6.27g (85%).
Step 1.4 2-butoxy-6-(5-pent-1-ynylpyrimidine-2-yl)naphthalene.
   Quantities: compound 1.3 (1.60g, 8.86mol), 6-butoxynaphth-2-ylboronic acid (2.60g, 0.011mol), tetrakis(triphenylphosphine)palladium(0) (0.35g, 0.30mol).
   This experimental procedure is a boronic acid coupling reaction. A solution of the boronic acid in dimethoxyethane is added to a stirred mixture of compound 1.3 and tetrakis(triphenylphosphine)palladium(0) in dimethoxymethane and 2M sodium carbonate at room temperature under dry nitrogen. The mixture is heated under reflux for 18 hours (glc analysis revealing a complete reaction). The crude product is purified by column chromatography [silica gel/petroleum fraction (bp40-60°C)-dichloromethane, 1:2] to give a colourless solid which is recrystallised from ethyl acetate-ethanol (1:1) to yield colourless crystals. Yield is 2.25g (74%).

### Example 2. (Comparative) Preparation of:

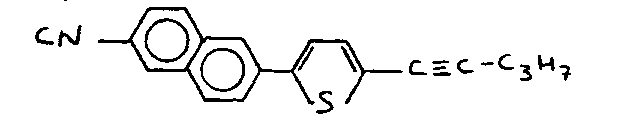

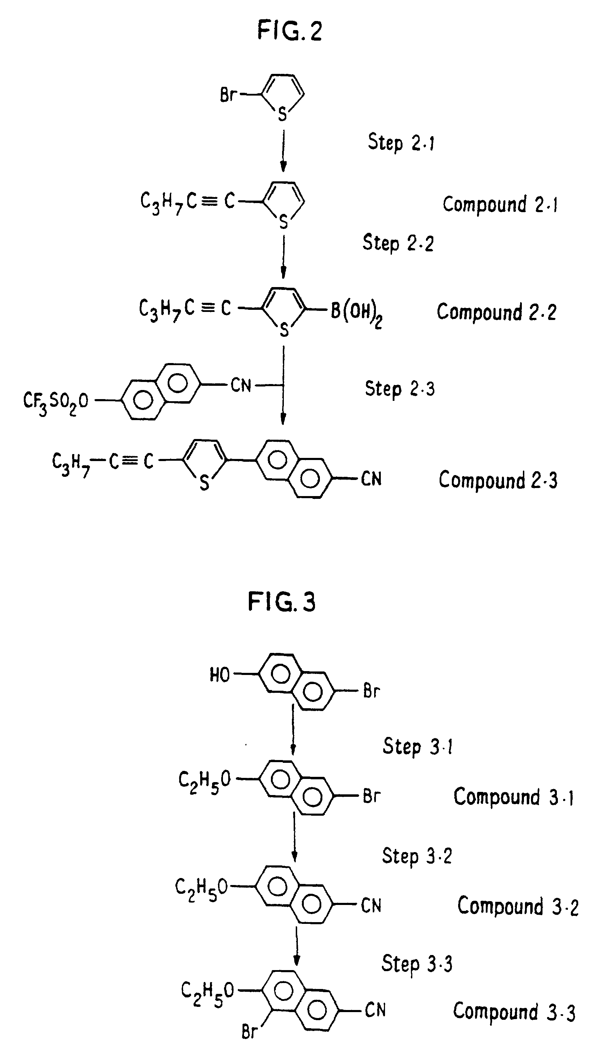

With reference to figure 2 it can be seen that
Step 2.1 2-pent-1-ynylthiophene.
   Quantities: pent-1-yne (6.80g, 0.10mol), n-butyllithium (10.0ml, 10.0M in hexane, 0.044mol), zinc chloride (13.60g, 0.10mol), 2-bromothiophene (16.00g, 0.098mol), tetrakis (triphenylphosphine)palladium(0) (3.40g 2.94mol).
   The experimental procedure is as described for step 1.3. The crude product is distilled to yield a colourless liquid. Yield is 12.96g (88%).
Step 2.2 5-pent-1-ynylthiophen-2-ylboronic acid.
   Quantities: compound 2.1 (10.00g, 0.067mol), n-butyllithium (6.80g, 10.0M in hexane, 0.068mol), trimethyl borate (14.20g, 0.137mol).
   This experimental procedure is a standard boronic acid preparation and yields a brown solid. The yield is 12.00g (93%).
Step 2,3 2-(6-Cyanonaphth-2-yl)-5-pent-1-ylthiophene.
   Quantities: 6-cyanonaphth-2-yl triflate (2.00g, 6.64mol), 5-pent-1 -ynylthiophen-2-ylboronic acid (1.55g, 7.99mmol), tetrakis (triphenyl phosphine)palladium(0) (3.40g 2.94mol), lithium chloride (0.85g, 0.020mol).
   This experimental procedure is as for the boronic acid coupling reaction described in step 1.4, except for the addition of lithium chloride as a catalyst. The crude product is purified by column chromotography [silica gel/ petroleum fraction (bp40-60°C) - dichloromethane, 1:1] to give a yellow solid, which is recrystallised from ethanol to yield pale yellow crystals. Yield is 1.55g (78%).

### Example 3. (Comparative) Preparation of:

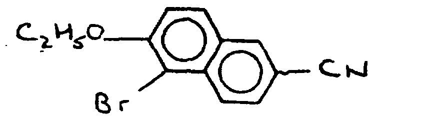

With reference to figure 3 it can be seen that
Step 3.1 2-Bromo-6-ethoxynaphthalene.
   This experimental procedure is an 0-alkylation. A solution of bromomethane (48.90g, 0.45mol) is added dropwise to a stirred refluxing mixture of 6-Bromo-2-naphthol (40.00g, 0.18mol) and potassium carbonate (63.00g, 0.46mol) in acetone. The stirred mixture is heated under reflux for 24 hours (ie until glc analysis reveals a complete reaction). The potassium carbonate is filtered off, water is added to filtrate and the product is recrystallised from ethanol to give a yield of 43.92g (97%).
Step 3.2 2-Ethoxy-6-cyanonaphthalene.
   This experimental procedure is a cyanation where compound 3.1 (3.00g, 0.012mol) is reacted with Copper (I) cyanide (1.30g, 0.015mol), with the crude product purified by column chromatography [silica gel/petroleum fraction (bp40-60°C) - dichloromethane, 1:2) to give a colourless solid which is distilled [Kugeklrohr, 170°C (maximum) at 0.1mmHg] to yield 1.75g (74%) of colourless solid.
Step 3.3 1-Bromo-6-cyano-2-ethoxynaphthalene.
   A solution of bromine (1.72g, 0.011mol) in glacial acetic acid (4ml) is added dropwise to stirred solution of compound 3.2 (1.93g, 9.80mol) in glacial acetic acid (35ml) at 80°C. The solution is stirred for an additional 10 minutes (glc revealing a complete reaction and only one product peak). The solution is added to water and the product is extracted from ether (x2) and the combined ethereal extracts are washed with water and sodium metabisulphite and dried. The solvent is removed *in vacuo* and the residue purified by column chromotography [silica gel/petroleum fraction (bp40-60°C) - dichloromethane, 1:1] to give a colourless solid (1.72g) which is recrystallised from hexane-dimethoxymethane (1:1) to yield 1.55g (57%) colourless crystals.

### Example 4. (Comparative) Preparation of

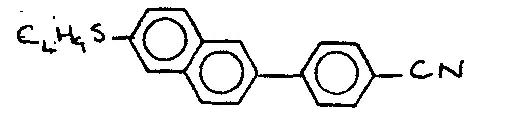

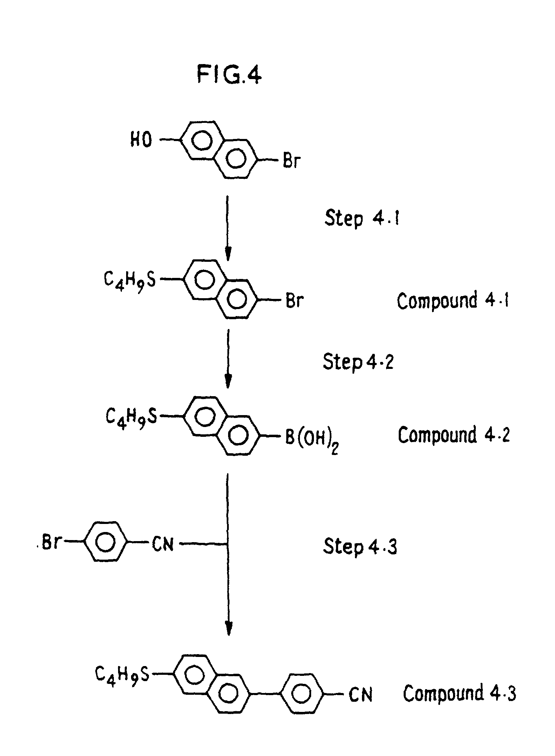

With reference to figure 4 it can be seen that
Step 4.1 2-Bromo-6-thiobutylnaphthalene.
   Trifluoromethanesulphonic acid (14.85g, 0.099mol) is added dropwise to a stirred mixture of 6-bromo-2-naphthol (20.00g, 0.090mol) and butanethiol (8.07g, 0.090mol) in dry benzene (180ml) under dry nitrogen. The resulting solution is stirred at 50°C for 4 hours (glc analysis revealing a complete reaction), cooled and poured into ice-cold water. The product is extracted into ether (x2), the combined organic phases washed with 5%sodium hydroxide and water and dried (MgSO₄). The solvent can then be removed *in vacuo* and the residue distilled to give a colourless solid. Yield is 13.80g (52%).
Step 4.2 6-Thiobutylnaphth-2-ylboronic acid
   Quantities: compound 4.1 (6.80g, 0.023mol), n-butyllithium (2.30ml, 10.0M in hexane, 0.023mol), trimethylborate (4.80g, 0.046mol).
   This experimental procedure is a preparation of the appropriate boronic acid by standard procedures, giving a yield of 3.49g (58%) after the product of the reaction is extracted into 10% potassium hydroxide, the basic extract washed with ether and acidified, extracted into ether and dried (MgSO₄), and removal of solvent *in vacuo* to give a colourless liquid.
Step 4.3 2-(4-Cyanophenyl)-6-thiobutylnaphthalene.
   Quantities: compound 4.2 (1.81g, 6.96mol),4-bromobenzonitrile (1.15g,6.32mol), tetrakis(triphenylphosphine)palladium(0) (0.235g, 0.22mol).
   This experimental procedure is a boronic acid coupling reaction as previously described in step 1.4, followed by purification of the crude product by column chromotography [silica gel/ petroleum fraction (bp40-60°C) - dichloromethane, 1:1] to give a colourless solid which is then recrystallised from ethanol-ethyl acetate (4:1) to yield 1.63g (81%) of colourless crystals.

### Example 5. (Comparative) Preparation of

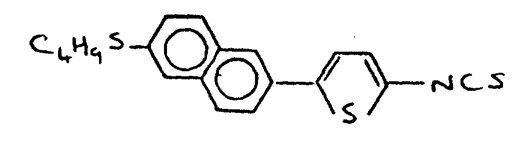

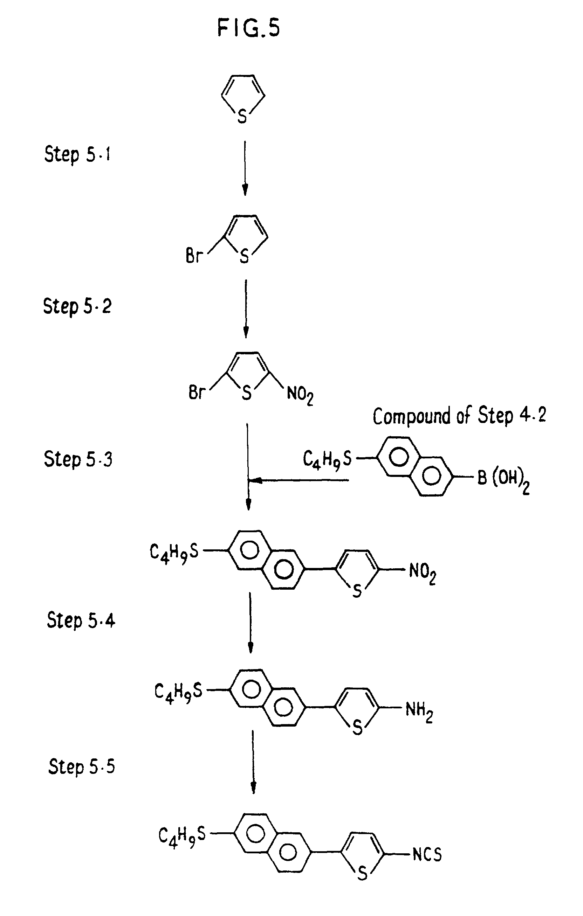

With reference to figure 5 it can be seen that
Step 5.1 2-Bromothiophene.
   A solution of thiophene (31.88g, 0.380mol) and N-bromosuccinimide (64.00g, 0.360mol) in a mixture of chloroform (80ml) and glacial acetic acid (80ml) was heated under reflux (with stirring) for 0.5 hours (constant glc analysis revealed a complete reaction with minimal formation of 2,5-dibromothiophene). The reaction mixture was diluted with water and washed with dichloromethane (2x100ml); the combined organic extracts were washed successively with water (300ml) and aqueous potassium hydroxide (5%, 300ml) before being dried (MgSO₄). The solvent was removed *in vacuo* and the residue was distilled to give a colourless liquid.
   Yield 24.34g (42%).
Step 5.2 2-Bromo-5-nitrothiophene.
   Nitric acid (24.00g, 1.42 sp gr. 0.381mol) in acetic anhydride (50ml) at 0°C was added dropwise to a cooled (0°C) rapidly stirred solution of the compound of step 5.1 (24.77g, 0.152mol) in acetic anhydride (50ml). At the end of the addition the stirring was continued for 0.5 hr and the mixture was refrigerated overnight. The mixture was poured into ice water (400ml) and the precipitate was filtered off, dissolved in ether (2x200ml), and washed with water until free of acid. The solvent was removed *in vacuo* and the residue was purified by column chromatography [silica gel/petroleum fraction (bp 40-60°C), dichloromethane, 5:1] and was recrystallised from ethanol/dimethoxyethane, 100:1 to give a pale yellow solid which was dried *in vacuo* (P₂O₅) to give 66% yield of 20.89g.
Step 5.3 2-(6-Thiobutyl-2-naphthyl)-5-nitrothiophene.
   Quantities: Compound of step 4.2 (4.00g, 0.015mol),compound of step 5.2 (3.31g, 0.016mol), tetrakis(triphenylphosphine)palladium(0) (0.910g, 0.001mol), sodium carbonate (15.8ml, 2.0M, 0.03mol).
   The compound of step 5.2 was added all at once to a rapidly stirred mixture of the palladium catalyst, the compound of step 4.2, and aqueous sodium carbonate solution in dimethoxymethane under dry nitrogen. The reaction mixture was refluxed overnight (tlc and glc revealed a complete reaction) and the product was extracted into ether; the combined ethereal solutions were washed with saturated sodium chloride solution and dried (MgSO₄). The solvent was removed *in vacuo* and the product was purified by column chromatography [silica gel/ petroleum fraction (bp 40-60), dichloromethane, 5:1] to give an orange solid with a 71% yield (3.41g).
Step 5.4 2-(6-thiobutyl-2-naphtyl)-5-aminothiophene.
   A stirred solution of compound of step 5.3 (2.62g, 0.008mol) and palladium on carbon (10%, 2.22g) in ethanol and tetrahydrofuran was stirred under hydrogen overnight. The catalyst was removed by filtration through "Hyflo supercel" and the solvent was removed *in vacuo* to afford a black solid which was used in the next step without purification after yielding 2.39g (100%), purity (glc) 50%.
Step 5.5 2-(6-thioputyl-2-naphthyl)-5-isothionatothiophene.
   A solution of compound of step 5.4 (2.30g, 0.007mol), in chloroform was added to a stirred, cooled (0-5°C) solution of calcium carbonate (1.17g, 0.012mol) and thiophosgene (0.97g, 0.008mol) in water and chloroform at 0-5°C. The mixture was heated at 35°C for 1h (glc and tlc analysis confirmed a complete reaction) and poured into water. The organic layer was washed with hydrochloric acid (1%, 100ml) and dried (MgSO₄). The compound was purified by column chromatography [silica gel/ petroleum (bp40-60°C), dichloromethane, 5:1] and was recrystallised to give 0.57g (22% yield) of a pale green solid which was dried *in vacuo* (CaCO₃), having an indicated (hplc) purity of >99%.

In the previous examples, Examples 1-5 are not covered by the scope of the present invention. Similarly in Figures 1-5 final compounds 1.4, 2.3, 3.3, 4.3, 5.5 are not covered by the scope of the invention. In Table I the sixth and seventh compounds, i.e. those containing a CO₂ linking group are covered by the scope of the present invention.

Liquid crystal transition temperatures between crystalline (K), nematic (N), smectic B (S_{B}) and isotropic (I) are given in Table 1 below for compounds of Formula I and Formula II. The table also contains a comparison of anisotropy in polarisability Δ a with that of 4-cyano-(4'pentyl)-1-phenylcyclohexane (5PCH) and the birefringence (Δn) of the compounds. [ ] denotes a virtual phase transition.

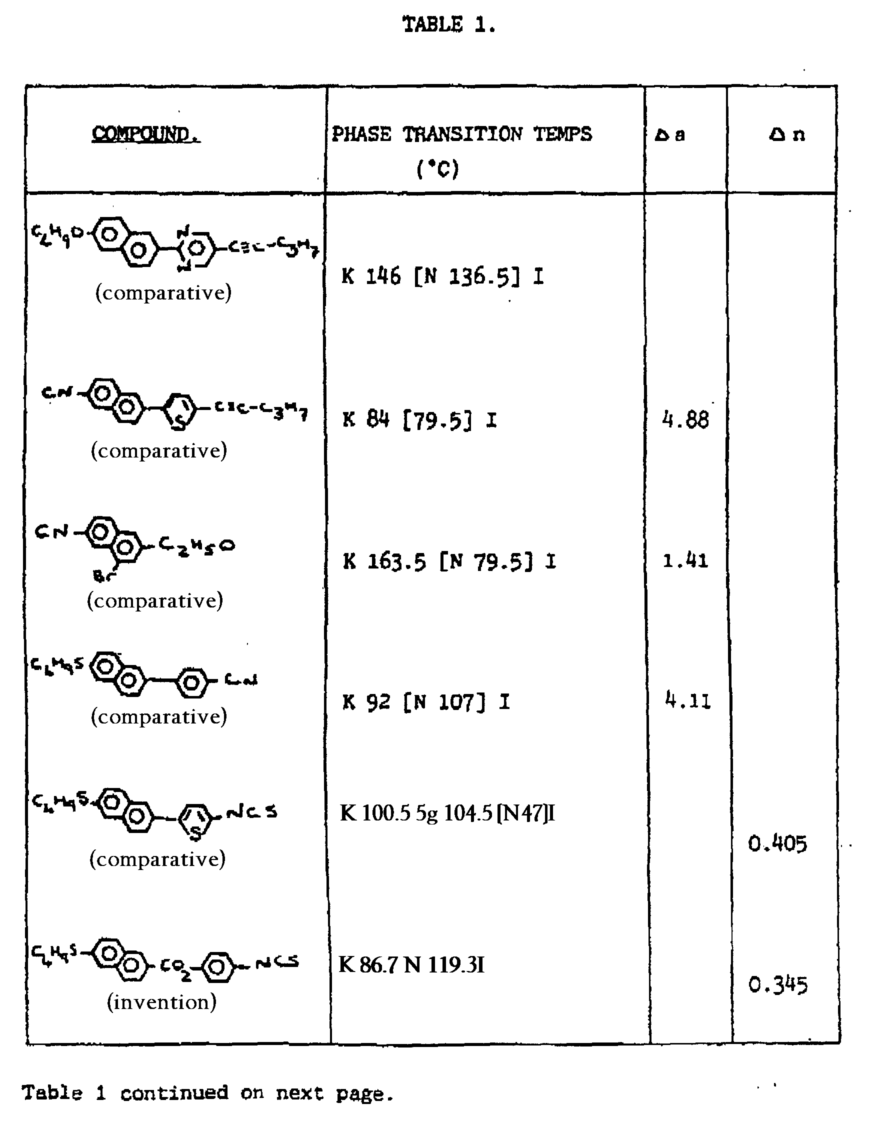

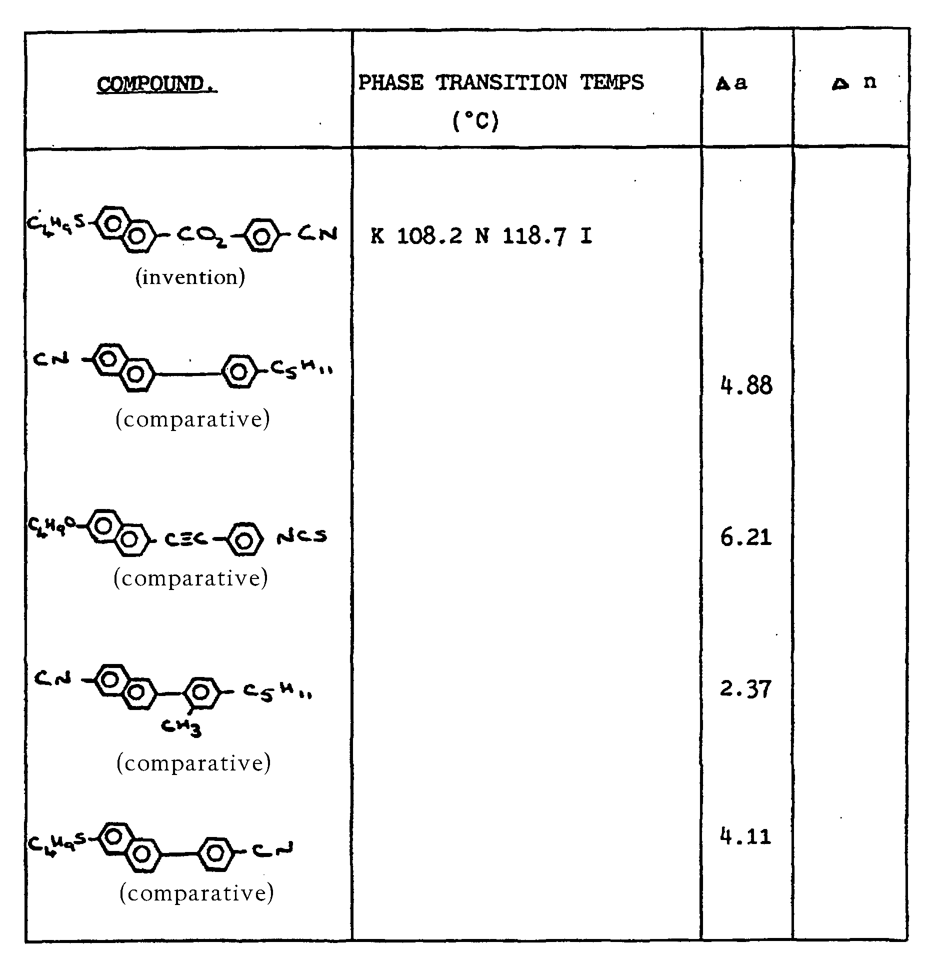

The Δn measurements are normalized for 25°C and were carried out using an Abbe refractometer and using 3 wt% of the compound to be measured in a non-polar eutectic nematic host, typically such as 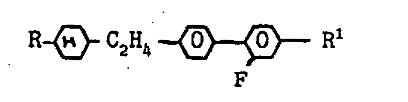 where R and R' are alkyl.

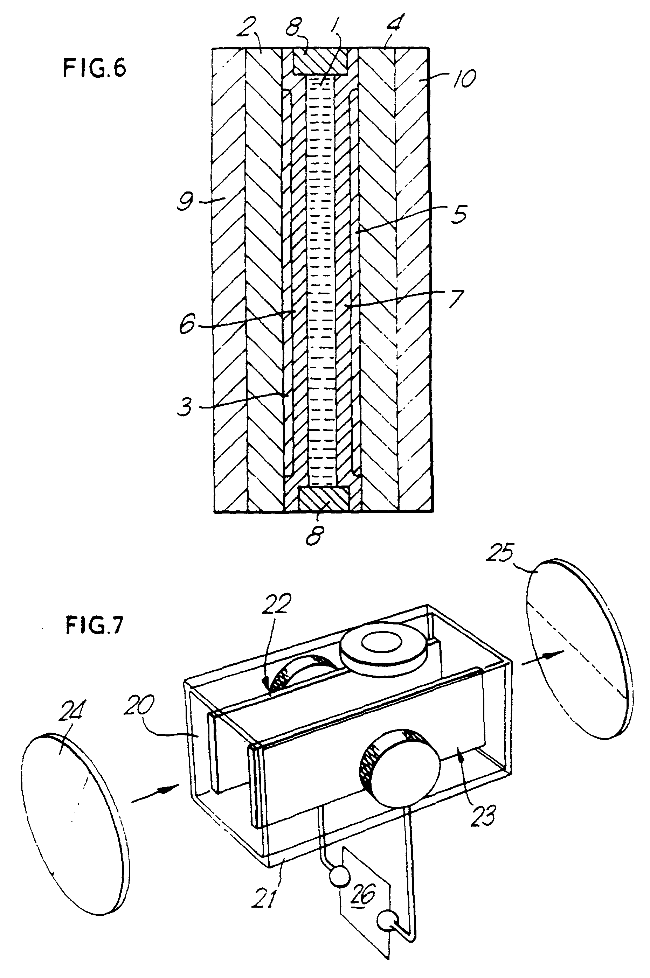

Figure 6 a liquid crystal cell comprises a layer 1 of liquid crystal material, where the material is a mixture incorporating compounds of formula I, sandwiched between a glass slide 2 having a conducting layer 3 on its surface, eg of indium tin oxide, and a glass slide 4 having a transparent conducting layer 5 on its surface. The slides 2,4 bearing the layers 3.5 are respectively coated with films 6.7 of a polyimide layer. Prior to construction of the cell the films 6 and 7 are rubbed with a soft tissue in a given direction, the rubbing directions being arranged parallel to the construction of the cell. A spacer 8 eg of polymethylmethacrylate, separates the slides 2,4 to the required distance eg 5 microns. The liquid crystal material 1 is introduced between the slides 2,4 by filling the space between the slides 2,4 and spacer 8 and sealing the spacer 8 in a vacuum in a known way.

A polarizer 9 is arranged with its polarization axis parallel to the rubbing direction on the films 6.7 and an analyzer (crossed polariser) 10 is arranged with its polarization axis perpendicular to that rubbing direction. When a voltage is applied across the cell by making contact with the layers 3 and 5 the cell is switched.

In an alternative device (not shown) based on a cell construction as shown in figure 6 the layers 3 and 5 may be selectively etched in a known way, eg by photoetching or deposition through a mask, eg to provide one or more display symbols, eg letters, numerals, words or graphics and the like as conventionally seen on displays. The electrode portions thereby may be addressed in a variety of ways which include multiplexed operation.

Figure 7 shows a Kerr cell 20. It comprises a glass cell 21 having two electrodes 22 and 23, which can be filled with a polar isotropic medium such as compounds of formula II or materials comprising mixtures including at least one compound of formula II. The cell 20 can be positioned between crossed linear polarisers 24 and 25, whose transmission axes are arranged to be at ± 45° to an applied electric field. Where there is zero voltage across the electrodes 22 and 23, and the cell 20 acts as a closed shutter. The application of a modulating electric field from voltage source 26 generates an electric field causing the cell 20 to act as a variable wave plate and thus operating as a variable aperture shutter where opening is proportional to the electric field.

## Claims

1. A liquid crystalline compound **characterised by** Formula 1
R₁―A―(X)ₘ ―(B)ₙ―R₂ Formula I
where A is naphthyl, B is selected from phenyl, methylated phenyl, brominated phenyl, fluorinated phenyl, thiophene, pyrimidine and pyridine, R₁ is thioalkyl, R₂ is selected from CN, SCN, NCS, X is COO, m is 1, n is 1.

2. A compound according to claim 1 where B is thiophene and A is naphthyl.

3. A compound according to claim 1 where B is pyrimidyl and A is naphthyl.

4. A liquid crystalline material, being a mixture of compounds, and **characterised in that** it comprises at least one compound according to claim 1.

5. A liquid crystalline material, being a mixture of compounds, and **characterised in that** it comprises at least one compound of claims 1 to 3

6. A material according to claim 4 and further **characterised in that** it comprises at least one compound of Formula III 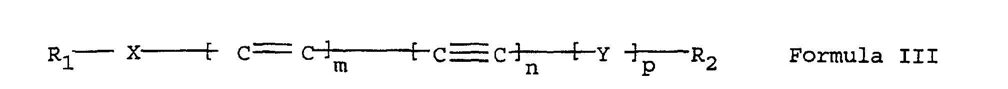 where R₁ is selected from a group comprising hydrogen, alkyl, alkoxy, alkynyl, thioalkyl, CN, and Br; R₂ is selected from a group comprising hydrogen, NCS, SCN, CN, alkyl, alkoxy, alkynyl and thioalkyl; m and n are 1 or 0 such that m is 1 where n is 0 and m is 0 where n is 1 or 0; p is independently 1 or 0; X is selected from a group comprising of naphthyl, fluorinated naphthyl and brominated naphthyl; and Y is selected from a group comprising of phenyl, methylated phenyl, brominated phenyl, thiophene and pyrimidine and pyridine.

7. A material according to claim 4 and further **characterised in that** it comprises at least one compound of Formula IV 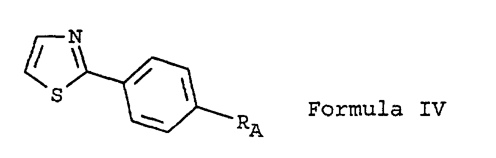 where R_{A} is selected from CN, alkyl and alkoxy.

8. A material according to claim 4 and further **characterised in that** it comprises at least one compound of Formula V 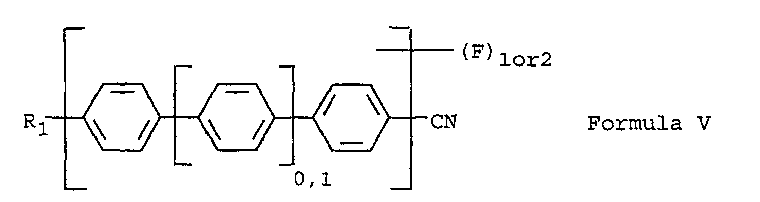 where R₁ is C₁₋₁₀ n-alkyl or n-alkoxy and the fluorosubstituent(s) may be in any one or two of the available substitution positions.

9. A liquid crystal device **characterised in that** it uses a material according to claim 4.

10. A liquid crystal device **characterised in that** it comprises a material according to claim 5.

11. Use of a liquid crystal compound according to any of claims 1-3 for inclusion in devices utilising pretransitional characteristics of liquid crystalline materials in the isotropic phase.

12. Use of a compound according to claim 11 where A is naphthyl and B is phenyl.

13. Use of a compound according to claim 12 where R₂ is CN.

14. Use of a liquid crystalline material according to claim 5 for inclusionin devices utilising pretransitional characteristics of liquid crystalline materials in the isotropic phase.

15. Use of a liquid crystalline material according to claim 14, wherein the following compounds may in addition or alternatively be present A is naphthyl, B is phenyl and/or A is naphthyl, B is phenyl, R₂ is CN.

16. Use of a material according to claim 14 and further **characterised in that** it comprises at least one compound of Formula III 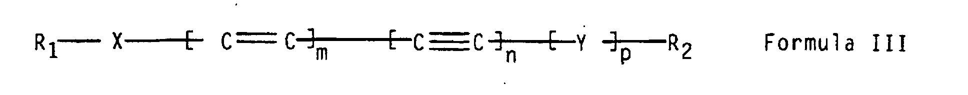 where R₁ is selected from a group comprising hydrogen, alkyl, alkoxy, alkynyl, thioalkyl, CN, and Br; R₂ is selected from a group comprising hydrogen, NCS, SCN, CN, alkyl, alkoxy, alkynyl and thioalkyl; m and n are 1 or 0 such that m is 1 where n is 0 and m is 0 where n is 1 or 0; p is independently 1 or 0; X is selected from a group comprising ofnaphthyl, fluorinated naphthyl and brominated naphthyl; and Y is selected from a group comprising of phenyl, methylated phenyl, brominated phenyl, thiophene and pyrimidine and pyridine.

17. Use of a material according to claim 14 and further **characterised in that** it comprises at least one compound of Formula IV 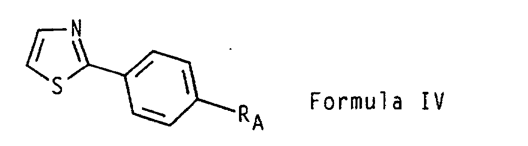 where R_{A} is selected from CN, alkyl and alkoxy.

18. Use of a material according to claim 14 and further **characterised in that** it comprises at least one compound of Formula V 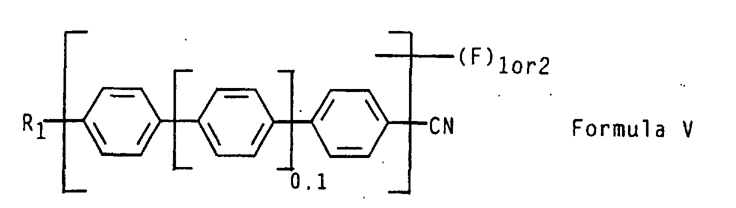 where R₁ is C₁₋₁₀ n-alkyl or n-alkoxy and the fluorosubstituent(s) may be in any one or two of the available substitution positions.

## Patentansprüche

1. Flüssigkristalline Verbindung, **gekennzeichnet durch** Formel 1:
R₁ ― A ― (X)ₘ ― (B)ₙ ― R₂ Formel I
worin A Naphthyl ist, B aus Phenyl, methylierten Phenyl, bromiertem Phenyl, fluoriertem Phenyl, Thiophen, Pyrimidin und Pyridin ausgewählt ist, R₁ Thioalkyl ist, R₂ aus CN, SCN, NCS ausgewählt ist, X COO ist, m gleich 1 ist, n gleich 1 ist.

2. Verbindung nach Anspruch 1, wobei B Thiophen ist und A Naphthyl ist.

3. Verbindung nach Anspruch 1, wobei B Pyrimidyl ist und A Naphthyl ist.

4. Flüssigkristallines Material, das ein Gemisch von Verbindungen ist und **dadurch gekennzeichnet ist, daß** es mindestens eine Verbindung nach Anspruch 1 umfaßt.

5. Flüssigkristallines Material, das ein Gemisch von Verbindungen ist und **dadurch gekennzeichnet ist, daß** es mindestens eine Verbindung nach einem der Ansprüche 1 bis 3 umfaßt.

6. Material nach Anspruch 4, und außerdem **dadurch gekennzeichnet, daß** es mindestens eine Verbindung der Formel III umfaßt: 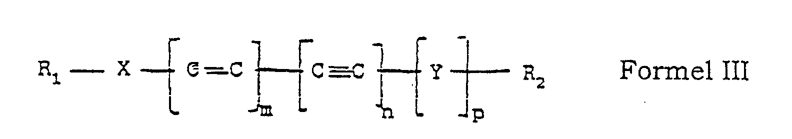 worin R₁ aus einer Gruppe, umfassend Wasserstoff, Alkyl, Alkoxy, Alkinyl, Thioalkyl, CN und Br, ausgewählt ist; R₂ aus einer Gruppe, umfassend Wasserstoff, NCS, SCN, CN, Alkyl, Alkoxy, Alkinyl und Thioalkyl, ausgewählt ist; m und n 1 oder 0 sind, so daß m 1 ist, wenn n 0 ist, und m 0 ist, wenn n 1 oder 0 ist; p unabhängig 1 oder 0 ist; X aus einer Gruppe, umfassend Naphthyl, fluoriertes Naphthyl und bromiertes Naphthyl, ausgewählt ist und Y aus einer Gruppe, umfassend Phenyl, methyliertes Phenyl, bromiertes Phenyl, Thiophen und Pyrimidin und Pyridin, ausgewählt ist.

7. Material nach Anspruch 4 und außerdem **dadurch gekennzeichnet, daß** es mindestens eine Verbindung der Formel IV umfaßt: 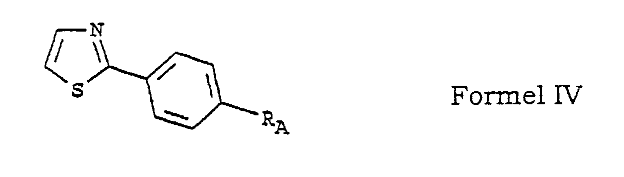 worin R_{A} aus CN, Alkyl und Alkoxy ausgewählt ist.

8. Material nach Anspruch 4 und außerdem **dadurch gekennzeichnet, daß** es mindestens eine Verbindung der Formel V umfaßt: 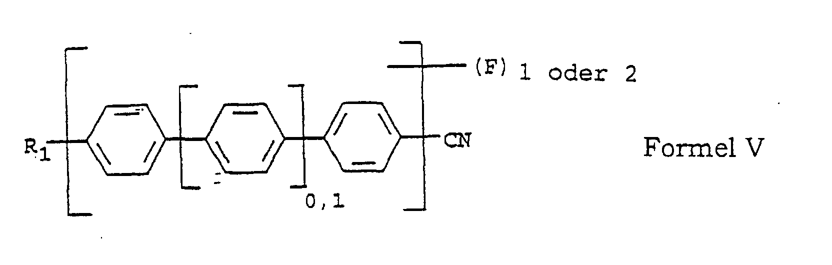 worin R₁ C₁₋₁₀-n-Alkyl oder n-Alkoxy ist und der Fluor-Substituent/die Fluor-Substituenten in irgendeiner oder zwei der verfügbaren Substitutionspositionen sein können.

9. Flüssigkristallvorrichtung, **dadurch gekennzeichnet, daß** sie ein Material nach Anspruch 4 verwendet.

10. Flüssigkristallvorrichtung, **dadurch gekennzeichnet, daß** sie ein Material nach Anspruch 5 umfaßt.

11. Verwendung einer Flüssigkristallverbindung nach einem der Ansprüche 1 bis 3 zum Einschluß in Vorrichtungen, die Vorübergangscharakteristika von flüssigkristallinen Materialien in der isotropen Phase ausnutzen.

12. Verwendung nach Anspruch 11, worin A Naphthyl ist und B Phenyl ist.

13. Verwendung nach Anspruch 12, worin R₂ CN ist.

14. Verwendung eines flüssigkristallinen Materials nach Anspruch 5 zum Einschluß in Vorrichtungen, die Vorübergangscharakteristika von flüssigkristallinen Materialien in der isotropen Phase ausnutzen.

15. Verwendung eines flüssigkristallinen Materials nach Anspruch 14, wobei die folgenden Verbindungen alternativ oder zusätzlich vorliegen können: A ist Naphthyl, B ist Phenyl und/oder A ist Naphthyl, B ist Phenyl, R₂ ist CN.

16. Verwendung eines Materials nach Anspruch 14 und außerdem **dadurch gekennzeichnet, daß** es mindestens eine der Verbindungen der Formel III umfaßt: 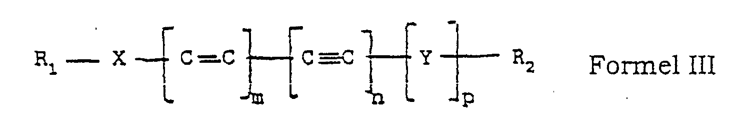 worin R₁ aus einer Gruppe, umfassend Wasserstoff, Alkyl, Alkoxy, Alkinyl, Thioalkyl, CN und Br, ausgewählt ist; R₂ aus einer Gruppe, umfassend Wasserstoff, NCS, SCN, CN, Alkyl, Alkoxy, Alkinyl und Thioalkyl, ausgewählt ist; m und n gleich 1 oder 0 sind, so daß m gleich 1 ist, wenn n gleich 0 ist, und m gleich 0 ist, wenn n gleich 1 oder 0 ist; p unabhängig 1 oder 0 ist; X aus einer Gruppe, umfassend Naphthyl, fluoriertes Naphthyl und bromiertes Naphthyl, ausgewählt ist; und Y aus einer Gruppe, umfassend Phenyl, methyliertes Phenyl, bromiertes Phenyl, Thiophen und Pyrimidin und Pyridin, ausgewählt ist.

17. Verwendung eines Material nach Anspruch 14 und außerdem **dadurch gekennzeichnet, daß** es mindestens eine Verbindung der Formel IV umfaßt: 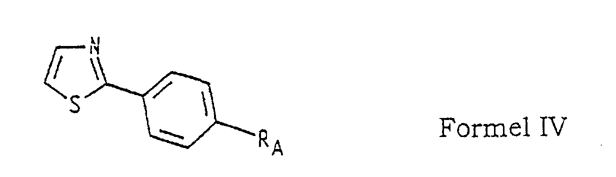 worin R_{A} aus CN, Alkyl und Alkoxy ausgewählt ist.

18. Verwendung eines Material nach Anspruch 14 und außerdem **dadurch gekennzeichnet, daß** es mindestens eine Verbindung der Formel V umfaßt: 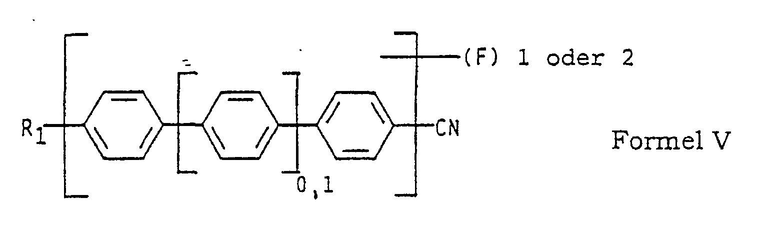 worin R₁ C₁₋₁₀-n-Alkyl oder n-Alkoxy ist, und der Fluor-Substituent/die Fluor-Substituenten in irgendeiner oder zwei der verfügbaren Substitutionspositionen sein kann/können.

## Revendications

1. Composé de cristaux liquides **caractérisé par** la Formule 1
R₁ ― A ― (X)ₘ ― (B)ₙ ― R₂ scan 1 Formule I
où A est un naphtyle, B est un phényle, un phényle méthylé, un phényle bromé, un phényle fluoré, du thiophène, une pyrimidine et une pyridine, R₁ est un thioalkyle, R₂ est CN, SCN, NCS, X est COO, m est égal à 1, n est égal à 1.

2. Composé selon la revendication 1, où B est un thiophène et A est un naphtyle.

3. Composé selon la revendication 1, où B est un pyrimidyle et A est un naphtyle.

4. Matériau de cristaux liquides qui est un mélange de composés, et **caractérisé en ce qu'**il comporte au moins un composé selon la revendication 1.

5. Matériau de cristaux liquides, qui est un mélange de composés, et **caractérisé en ce qu'**il comporte au moins un composé selon les revendications 1 à 3.

6. Matériau selon la revendication 4, et caractérisé de plus en ce qu'il comporte au moins un composé ayant la Formule III 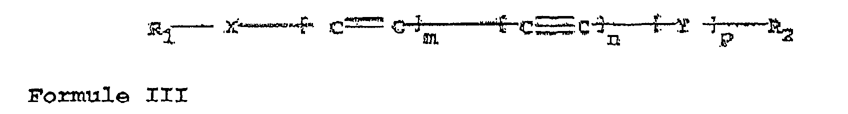 où R₁ est sélectionné parmi le groupe constitué d'hydrogène, d'alkyle, d'alkoxy, d'alcynyle, de thioalkyle, de CN et Br, R₂ sélectionné parmi un groupe constitué d'hydrogène, de NCS, de SCN, de CN, d'alkyle, d'alkoxy, d'alcynyle et thioalkyle, m et n sont égaux à 1 ou 0 de sorte que m est égal à 1 lorsque n est égal à 0 et m est égal à 0 lorsque n est égal à 1 ou 0, p est indépendamment égal à 1 ou 0, X est sélectionné parmi un groupe constitué de naphtyle, de naphtyle fluoré et de naphtyle bromé, et Y est sélectionné parmi un groupe constitué de phényle, de phényle méthylé, de phényle bromé, de thiophène et de pyrimidine ou et de pyridine.

7. Matériau selon la revendication 4, et caractérisé de plus en ce qu'il comporte au moins un composé ayant la Formule IV 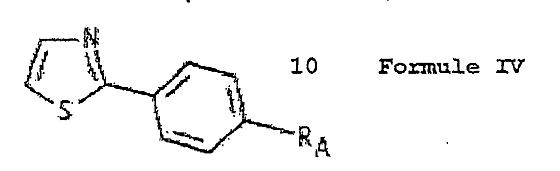 où R_{A} est sélectionné parmi CN, un alkyle et un alkoxy.

8. Matériau selon la revendication 4, et caractérisé de plus en ce qu'il comporte au moins un composé ayant la Formule V 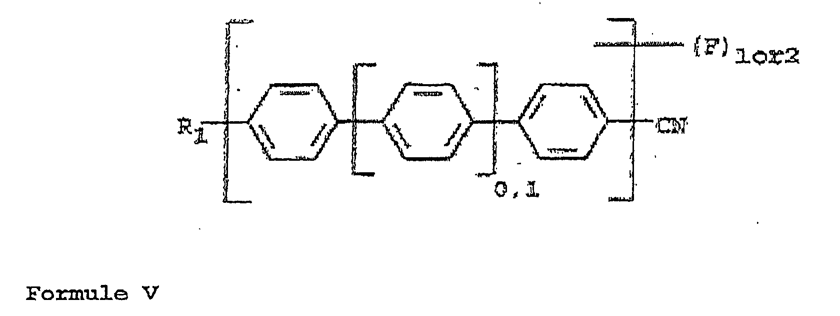 où R₁ est un n-alkyle ou un n-alkoxyle C₁₋₁₀ et le ou les substituants fluorés peuvent être à l'une quelconque des positions de substitution disponibles, ou à deux de celles-ci.

9. Dispositif à cristaux liquides **caractérisé en ce qu'**il utilise un matériau selon la revendication 4.

10. Dispositif à cristaux liquides **caractérisé en ce qu'**il comporte un matériau selon la revendication 5.

11. Utilisation d'un composé de cristaux liquides selon l'une quelconque des revendications 1 à 3 pour introduction dans des dispositifs utilisant des caractéristiques pré-transitionnelles de matériau de cristaux liquides dans la phase isotrope.

12. Utilisation d'un composé selon la revendication. 11, où A est un naphtyle et B est un phényle.

13. Utilisation d'un composé selon la revendication 12, où R₂ est CN.

14. Utilisation d'un matériau de cristaux liquides selon la revendication 5, pour introduction dans des dispositifs utilisant des caractéristiques pré-transitionnelles de matériaux de cristaux liquides dans la phase isotrope.

15. Utilisation d'un matériau de cristaux liquides selon la revendication 14, dans lequel les composés suivants peuvent de plus ou en variante être présents, A est un naphtyle, B est un phényle et/ou A est un naphtyle, B est un phényle, R₂ est CN.

16. Utilisation d'un matériau selon la revendication 14, et caractérisé de plus en ce qu'il comporte au moins un composé ayant la Formule III 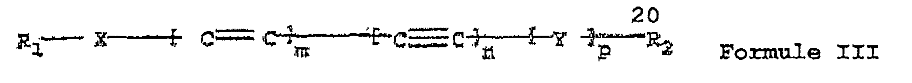 où R₁ est sélectionné parmi le groupe constitué d'hydrogène, d'alkyle, d'alkoxy, d'alcynyle, de thioalkyle, de CN et Br, R₂ sélectionné parmi un groupe constitué d'hydrogène, de NCS, de SCN, de CN, d'alkyle, d'alkoxy, d'alcynyle et thioalkyle, m et n sont égaux à 1 ou 0 de sorte que m est égal à 1 lorsque n est égal à 0 et m est égal à 0 lorsque n est égal à 1 ou 0, p est indépendamment égal à 1 ou 0, X est sélectionné parmi un groupe constitué de naphtyle, de naphtyle fluoré et de naphtyle bromé, et Y est sélectionné parmi un groupe constitué de phényle, de phényle méthylé, de phényle bromé, de thiophène et de pyrimidine ou et de pyridine.

17. Utilisation d'un matériau selon la revendication 14, et caractérisé de plus en ce qu'il comporte au moins un composé ayant la Formule IV 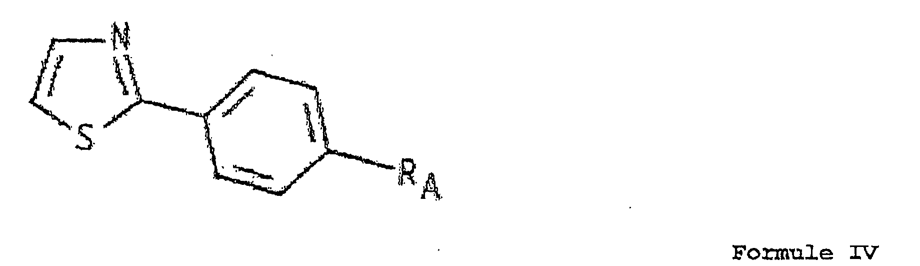 où R_{A} est sélectionné parmi CN, un alkyle et un alkoxy.

18. Utilisation d'un matériau selon la revendication 14, et caractérisé de plus en ce qu'il comporte au moins un composé ayant la Formule V 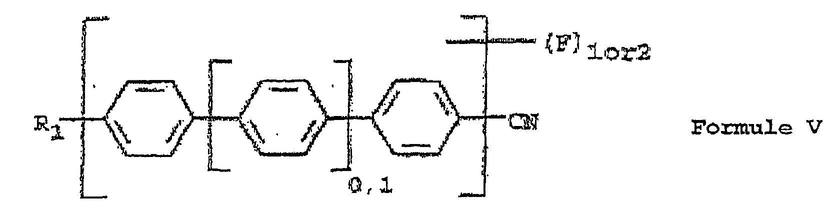 où R₁ est un n-alkyle ou un n-alkoxyle C₁₋₁₀ et le ou les substituants fluorés peuvent être à l'une quelconque des positions de substitution disponibles, ou à deux de celles-ci.
